# EUROPEAN PATENT APPLICATION

(11) **EP 2 641 973 A2**
(43) Date of publication of application: **25.09.2013**
(21) Application number: 11793467.9
(22) Date of filing: 19.09.2011
(51) Int. Cl.: C12Q 1/04, C12Q 1/70, G01N 33/569

(54) **METHOD AND ASSOCIATED SYSTEM FOR DETECTING AND ANALYSING PATHOGENIC AGENTS AND/OR AGENTS CAPABLE OF CAUSING THE DETERIORATION OF FOOD OF VEGETABLE ORIGIN**

(30) Priority: 15.11.2010 ES 201031680
(71) Applicant: Consejo Superior De Investigaciones Científicas (CSIC), 28006 Madrid (ES)
(72) Inventor: GIL MUÑOZ, María Isabel, E-30100 ESPINARDO (Murcia) (ES); SELMA GARCÍA, Victoria, E-30100 ESPINARDO (Murcia) (ES); LÓPEZ-GALVEZ, Francisco, E-30100 ESPINARDO (Murcia) (ES); ALLENDE PRIETO, Ana, E-30100 ESPINARDO (Murcia) (ES); BELTRÁN RIQUELME, David, E-30100 ESPINARDO (Murcia) (ES)
(74) Representative: Ungria López, Javier
(86) International application number: PCT/ES2011/070651
(87) International publication number: WO 2012/066165

(57) **Abstract**

The present invention relates to a method for the detection of potentially pathogenic agents and/or capable of causing deterioration in a plant sample, representative of one or several batches of plant product, characterized in that it comprises the concentration of a plant sample by centrifugation and the analysis of the presence or amount of potentially pathogenic agents and/or capable of causing deterioration in the concentrated sample obtained. Likewise, the present invention relates to a system for detection and analysis of potentially pathogenic agents and/or capable of causing deterioration in a plant sample to carry out said method.

## Description

The present invention relates to the field of biotechnology and relates to a method for the detection of pathogens and/or agents capable of causing deterioration in plant foods and a system that carries out said method, which allow improving biological safety and preserving the quality of plant foods by the detection of pathogens and/or microorganisms capable of causing deterioration of the same.

### STATE OF THE ART

The requirements relative to Good Agricultural Practices (GAP), Good Manufacturing Practices (GMP) and Good Distribution Practices (GDP) have as an object to minimize the risk of contamination of fresh-processed prepared products, which are ready to eat or to be cooked. The implementation of sanitation programs is necessary to ensure the safety of fruits and vegetables. Despite the progresses that are occurring in the field to reduce risks of contamination, these horticultural products have been involved in some problems related to public health.

The fresh-processed foods are fresh, washed, cut and packaged vegetable, fruits and garden produce products, ready for consumption. These fresh-processed products do not present in their processing any step to ensure their safety and it is through the use of GAP and GMP, that contamination by pathogenic microorganisms in food can be prevented.

Guidelines on quality and safety of fruit and garden produce in fresh-processed, specify the need for a washing or sanitizing step able to remove dirt, pesticide residues as well as microorganisms that cause loss of quality and food deterioration. Do not forget that, in the steps of elaboration of plant products in fresh-processed, procedures that can ensure complete asepsis are not employed, as it would be the case of the use of thermal treatments. Therefore, control of the micro flora will only be achieved through proper hygiene during elaboration steps and adequate conservation in modified atmosphere under refrigeration conditions.

In order to ensure the safety of consumption of these foods is necessary to check the absence of pathogens prior to distribution to consumers. The incidence of human illness associated with consumption of fresh produce has increased over the past two decades. The identification of pathogens in food and the environment has increasingly become a necessity rather important. Although there are many methods of detection available, food microbiologists often must choose between quantitative and identification methods without the possibility of combining both. Quantitative methods are generally based on the ability of viable bacterial cells of multiply in a medium rich in nutrients, although sometimes selective agents are added to support the growth of a specific group of organisms, such as coliform or enterococci. These methods that require an enrichment culture are relatively non-specific, since they quantify the total number of organisms belonging to one or more families in the analyzed sample. The non-specific methods most commonly used are both quantitative and semi quantitative, as the plate count method (e.g. counting aerobic microorganisms, coliform, yeast and fungi in plate), bioluminescence assays and impedance or conductance measurements.

Stevens and Jaykus (Critical Reviews in Microbiology, 2004, 30 (1): 7-24) describe the most frequently used methods for separation, concentration and identification of pathogens in food, and their advantages and disadvantages. Among other methods, these authors describe centrifugation as a widely used method for separation of microorganisms of the food product and their concentration with a view to their analysis. For the detection of pathogenic microorganisms in alfalfa sprouts and irrigation water, Johnston et. al. describe a method where the microorganisms are concentrated by centrifugation, DNA extraction is carried out and said microorganisms are detected and identified by PCR (Journal of Food Protection, 2005. Vol 68, 11, 2256-2263). Kumar et. al. describe a method for rapid detection of *Salmonella typhi* based on immunomagnetic separation and PCR. This method uses rinse water from the plants, which is centrifuged to obtain the microorganisms, the DNA of which is extracted for the detection of *S. typhi* by PCR (World Journal of Microbiology & Biotechnology. 2005, 21 (5):625-628).

US7691602 describes a method for rapid identification of microorganisms in an agricultural sample. This method allows rapid and efficient identification of pathogenic microorganisms without the need to enrich the sample by culture. It also allows determining the presence of statistically significant amounts of microorganisms in a whole crop. The method described in this patent is based on the analysis of preferably liquid samples through various consecutive filtrations. This method consists of passing large amounts of sample, such as the water from the plant washing tank, through various filters. The microorganisms retained on the filter are recovered by applying a pressure gas on the filter in the opposite direction of the filtering of the sample. After successive steps of filtration and recovery of microorganisms retained on the filter, the pathogens are identified by well known techniques such as immunoassays, PCR, culture, mass spectrometry and other.

WO2009111389 describes a method and an equipment, the OmniFresh™ 1000 from Hanson Technologies, to carry out this method that allows analyzing the water from the plant washing tank and detecting the presence of pathogenic microorganisms.

The optimization of a rapid, sensitive and effective method for the detection of pathogens in these plant foods is necessary to ensure their safety. Currently, most procedures intended to verify the biological quality of plant foods require an enrichment culture step and take more than 8 hours, except for the method described by Hanson Technologies, which allows identifying pathogens in about two hours from the sample collection.

### BRIEF DESCRIPTION OF THE INVENTION

A first aspect of the present invention relates to a method for the detection of potentially pathogenic agents and/or agents capable of causing deterioration of the plant in a plant sample, representative of at least one batch of plant product, characterized in that it comprises the following steps:
a. concentration of a plant sample by centrifugation,
b. analysis of the presence or the amount of potentially pathogenic agents in the concentrated sample obtained in (a).

A second aspect of the present invention relates to a system for detection and analysis of potentially pathogenic agents and/or agents capable of causing deterioration of the plant in a plant sample to carry out the method of the first aspect of the invention.

The following figures are provided by way of illustration and are not intended to be limiting of the present invention.

### BRIEF DESCRIPTION OF THE FIGURES

**FIG 1****.** Schematically illustrates the various steps in the chain of plant food processing as well as the various points at which samples were obtained for comparative studies of the present invention.
**FIG 2****.** Illustrates the standard curve for quantification by RT-PCR of E. *coli* 0157: H7 using the commercial kit from Applied Biosystems TaqMan® *E*. *coli* 0157: H7.

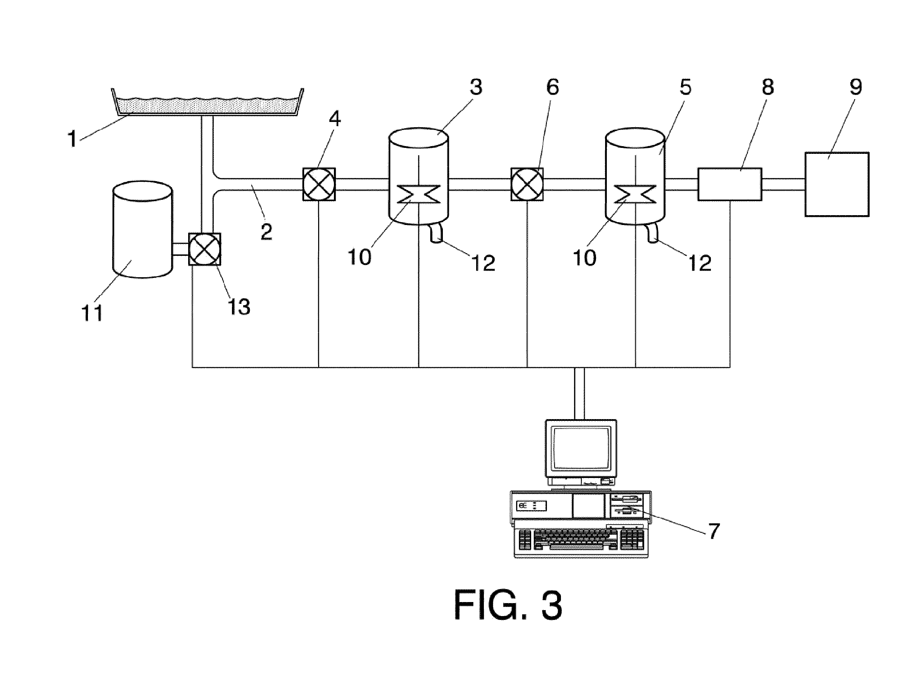

**FIG 3****.** Illustrates an embodiment of the system for detection and analysis of potentially pathogenic agents in a plant sample of the invention.

### DETAILED DESCRIPTION OF THE INVENTION

The present invention relates to a novel method to ensure biological safety of plant products intended for food and to a system for carrying out said method.

A first aspect of the invention is a method for direct and effective analysis of pathogens or agents capable of causing deterioration of the plant, by assessing the water after washing the plant destined for consumption in both fresh and frozen, canned or for being prepared as fresh-processed product, as well as a device for carrying out said procedure.

The present invention proposes the indirect analysis of one or several batches of plant product by the use of the remaining water in the plant after washing and rinsing thereof, wherein said water is recovered through a process of active drainage or drying, preferably by centrifugation or forced cold air tunnel. The fact of taking samples from the remaining water after washing through a process of active drainage or drying makes this method more sensitive to the recovery and detection of pathogens, as this process facilitates the dragging of those pathogens that are attached to the surface of the plant after the sanitation process.

The authors of the present invention have shown that the use of the plant active draining or drying water for the detection and/or quantification of the presence of potentially pathogenic agents is much more efficient than the use of the water from the washing tank or rinse water.

The authors of the present invention have comparative data on the efficiency of the procedure depending on the source of the sample: wash water, rinse water or centrifuge active draining water, also called centrifugal water. These experimental data show that the centrifugal water used as such or concentrated, is vital to the effectiveness of the procedure and that, therefore, the point of the processing line in which the sample is obtained is not indifferent.

Concentration of the water samples by centrifugation allows discarding the partially or completely used bacteria, as well as the free microbial DNA, reducing the risk of false positives. The method of the present invention allows therefore reducing the number of false positives due to detection of traces of pathogens, such as their DNA, that are not from viable, infectious organisms.

A second aspect of the present invention is a system for the detection and analysis of potentially pathogenic agents or agents capable of causing deterioration of the plant, in a plant sample, which allows carrying out the method of the first aspect of the present invention.

Therefore, a first aspect of the present invention relates to a method for the detection of potentially pathogenic agents and/ or agents capable of causing deterioration of the plant in a plant sample, representative of at least one batch of plant product, characterized in that it comprises the following steps:
a. concentration of a plant sample by centrifugation,
b. analysis of the presence or the amount of potentially pathogenic agents in the concentrated sample obtained in (a).

The term "potentially pathogenic agents" as used herein, refers to any organism, molecule or substance that may be infectious or harmful to health. Potentially pathogenic agents most commonly found in vegetables are bacteria, fungi, viruses and protozoa.

The term "agents capable of causing deterioration in the plant" as used herein, refers to any organism, molecule or substance likely to affect the organoleptic properties of the plant food.

The term "plant sample", as used herein, refers to a part or portion taken from one or more batches of plant product by methods that allow considering it as representative of it. The sample may be aqueous, without being water from the plant tissue itself, but it may be water that has been in contact with the surface of the plant.

Some of the potentially pathogenic agents that can be detected and quantified in the analysis of step (b) can be, but not limited to: *Escherichia coli, Salmonella* spp., *Listeria monocytogenes* and *Listeria* spp., *Staphyloccus* spp. or any other potentially pathogenic agent or indicator of contamination as well as agents capable of causing deterioration of the plant such as *Pseudomona* spp, *Lactobacillus* spp., *Pectobacterium* spp., *Alternaria* spp., *Botrytis* spp.

In a preferred embodiment of the present invention, the plant sample includes water from the industrial washing of the plant. Preferably, the plant sample is obtained during the active drainage or drying process of the plant. The process of active drainage or drying of the plant can be accomplished by any known technique. Preferably, the process of active drainage or drying of the plant is performed by centrifugation or forced cold air tunnel. The active drainage is defined as opposed to passive drainage that occurs simply by gravity from the plant washing in the processing chain, and relates to the process of removing from the plant surface the wash and/or rinse water by physical means such as centrifugation or application of an air flow to push any remaining water from the plant surface, as is the forced cold air tunnel. In the case of frozen plant products, a vibrating tray can be used to collect the water from the plant surface.

The centrifugation process for active drainage or drying of the plant is usually carried out at speeds around 225 *g* for a time of about 1 minute. In the process of active drainage or drying by forced cold air tunnel, a stream of cold air is applied such that the temperature of the plant product when it enters the tunnel is between 10 and 15 °C, and at the exit is between 2 and 3 °C. During the process of active drainage or drying in the forced cold air tunnel, for about 600 kg/hour is possible to eliminate about 90 liters of water.

In a preferred embodiment of the present invention, between 10 and 200 liters of water from plant industrial washing for every ton of plant product are obtained. The number of sampling to be performed will depend on the kilograms of processed plant product each day. A sampling equivalent to about 1,000 kg of plant per hour is typical.

In a preferred embodiment of the present invention, the centrifugation of step (a) is performed in one or more of a centrifugation step sequentially. Preferably, the centrifugation of step (a) is performed in two sequential steps.

When performing two or more sequential steps of centrifugation, the pellet obtained in the first centrifugation is resuspended, and this new concentrated sample is centrifuged in the next centrifugation step, and so forth in the following centrifugations. Thus, the sample is concentrated inversely proportional to its volume. For example, when a 250 ml volume plant sample is centrifuged and the pellet obtained is resuspended in a volume of 1 ml, it is said that the sample has been concentrated 250 times. If successive centrifugations are performed, the times the sample is concentrated in the first centrifugation are multiplied by the times the sample is concentrated in the second and successive centrifugations.

In a preferred embodiment of the present invention, the centrifugation of step (a) allows the concentration of the sample between 100 and 15,000 times. Preferably, in the first centrifugation step the sample is concentrated between 100 and 500 times, and in the second centrifugation step, the sample is concentrated, in addition, between 5 and 25 times.

In a preferred embodiment of the present invention, the centrifugation of step (a) is performed at a speed of between 2,000 *g* and 20,000 *g* for a time between 2 and 25 minutes. Preferably in step (a) is carried out a first centrifugation at a speed of 2,000 *g* and 5,000 *g* for a time between 5 and 20 minutes and a second centrifugation at a speed of between 12,000 *g* and 20,000 *g* for a time between 2 and 5 minutes.

In a preferred embodiment of the present invention, the centrifugation of step (a) allows the concentration of potentially pathogenic agents and/or agents capable of causing deterioration of the plant. Said centrifugation allows concentrating agents comprising at least one envelope and does not allow concentrating free DNA. In this way, obtaining false positives is prevented, i.e., results that indicate the presence of infectious agents and hazardous to the safety of the plant food, when there was really only DNA traces of such agents in the sample, and therefore not actual infectious agents.

In a preferred embodiment of the present invention up to 3 colony forming units (CFU) are detected per gram of plant product. A CFU is the minimum number of separable cells on the surface, or within, a semi-solid agar medium that results in the development of a visible colony on the order of tens of millions of progeny cells. A CFU can correspond to a pair, a chain or a cluster, as well as to a single cell.

In a preferred embodiment of the present invention, the analysis of step (b) comprises the extraction of DNA from potentially pathogenic agents present in the sample. Preferably, the analysis of step (b) is carried out by real time PCR (polymerase chain reaction) or any other method for rapid analysis of pathogens and/or agents capable of causing deterioration of the plant food.

In a preferred embodiment of the present invention, the duration of the two steps (a) and (b) is between 1.5 hours and 4 hours. Preferably, the duration of the two steps (a) and (b) is less than 2 hours.

A second aspect of the present invention relates to a system for detection and analysis of potentially pathogenic agents and/or agents capable of causing the deterioration of the plant in a plant sample to carry out the method of the first aspect of the invention, that comprises an analyzer (9) for the detection and quantification of potentially pathogenic agents and/or agents capable of causing the deterioration of the plant, characterized in that it additionally comprises:
- a first tank (3) for collecting the water driven into said first tank from a container (1) for collecting the water removed from the plant surface during the active drainage or drying process through a conduit (2), by first driving means (4),
- a second tank (5) for collecting a sample of water, driven into said second tank from the first tank (3) by second driving means (6),
- centrifugation means (8) for concentrating the potentially pathogenic agents present in the water, and
- a control system (7) for regulating: the flow rate driven by the first driving means (4); the flow rate driven by the second driving means (6), and the centrifugation process,
where the analyzer is adapted to analyze the centrifuged water.

The water from the active drainage or drying of the plant collected in the container (1) is free of plant debris. These debris can be removed by a filter or any other mechanism known in the state of the art.

In a preferred embodiment of the present invention, the first tank (3) and/or the second tank (5) comprise stirring means (10) for the homogenization of the water.

The first tank (3), called homogenization tank, is designed to collect all the water from the active drainage or drying of the plant, collected at different centrifugations or as the plant product passes through the forced cold air tunnel. All the water collected in the container (1) is transferred to the first tank (3) by the first driving means (4). In this way, it can be chosen if you want the first tank (3) to store the water from the active drainage or drying from a single batch or from more than one batch of plant product.

Thanks to the second driving means (6), part of the water from the first tank (3) is transferred to the second tank (5), called sampling tank.

In a preferred embodiment of the present invention, the first tank (3) and the second tank (5) comprise drainage systems (12) for emptying said tanks (3) and (5).

In the second tank (5) water samples from different fillings of the first tank (3) are mixed. Once the desired amount of water has been transferred from the first tank (3) to the second tank (5) by the second driving means (6), said first tank (3) is drained by a drainage system (12).

Once the desired amount of water has been transferred from the second tank (5) to the centrifugation means (8), said second tank (5) is drained through the drainage system (12).

In a preferred embodiment of the present invention, the system further comprises a disinfection unit (11) to provide a disinfectant solution to the conduit (2) by third driving means (13). The disinfectant solution may be an acid such as lactic acid or a base such as sodium hypochlorite, or it may be any other known disinfectant solution, such as a solution obtained by exposing an oxidant such as hydrogen peroxide to ultraviolet light, ozone or ultrasound, or using any other technology of disinfection or disinfectant. Once the disinfectant solution enters the conduit (2), it flows throughout the system due to the first and second driving means (4 and 6) to the second tank (5).

In another preferred embodiment of the present invention, at least one of the following elements: the stirring means (10) of the first tank (3) and the second tank (5); the drainage systems (12) for draining the first tank (3) and the second tank (5); the third driving means (13) for the application of the disinfectant solution of the disinfection unit (11) to the conduit (2), is regulated by the control system (7).

In a preferred embodiment of the present invention, the centrifugation means (8) comprise two different sets of collecting tubes for sequential centrifugation of the samples in two different steps so that in the second step the pellet from the first step is centrifuged.

Throughout the description and claims the word "comprise" and its variants are not intended to exclude other technical features, additives, components, or steps. For those skilled in the art, other objects, advantages and features of the invention will become apparent in part from the description and in part from the practice of the invention. The following examples are provided by way of illustration and are not intended to be limiting of the present invention.

### EXAMPLES

Next the invention is illustrated by means of tests conducted by the inventors that demonstrate the effectiveness of the method of the invention for the detection of potentially pathogenic agents in a plant sample as well as of the associated system.

### Example 1: The efficiency of the detection method depends on the point of the processing chain where the sample is taken.

The centrifugal water samples are processed in the laboratory at refrigeration temperature. Normally pathogenic bacteria that can contaminate the plant material are in very low concentration. Therefore, the concentration of these pathogenic bacteria that pass from the product to the wash and centrifugal water might be very low, being below the detection limit for extremely sensitive techniques, such as RT-PCR *("Real Time Polymerase Chain Reaction",* also called "quantitative ").

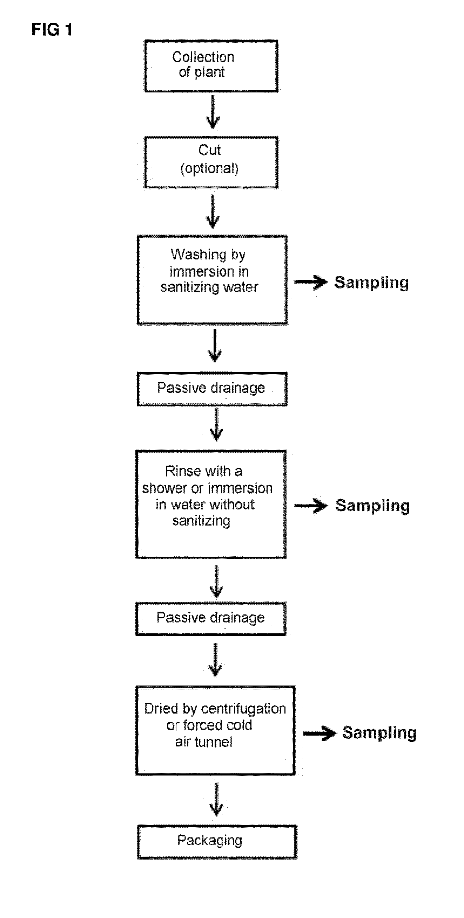

During the processing of fresh processed vegetables (fruits and garden produce minimally processed, chopped, washed and packaged) are carried out pre-cooling operations of raw materials, removing outer leaves, peeling and cutting. Then, the plants are subjected to a washing step in order to remove exudates and sanitize the product. Said step that has a duration of 0.5-2 minutes, is usually performed in a continuous washing tunnel with water and a sanitizer at temperatures of 4-6 °C. Many sanitizers require subsequent rinsing with water, which may be by immersion or shower and which should not last more than 1 min. Subsequently, excess water accumulated on the surface during washing is removed by centrifugation or forced cold air tunnel (Fig. 1). The system used will depend on the product characteristics such as sensitivity to mechanical damage, the morphology of the tissue, and so on. Subsequently, the food is packaged using active or passive atmosphere, taking into account the particularities of each plant (respiration rate, browning, etc.).

The detection and analysis of pathogen *Escherichia coli* 0157:H7 in vegetables was carried out, specifically in iceberg lettuce, in water samples collected at different places along the industrial processing line of the plant (Fig. 1):
- water from the wash tank,
- rinse water,
- active drainage or drying water (in this case by centrifugation).

A cocktail of 5 strains of *Escherichia coli* 0157:H7 from the Spanish Type Culture Collection was used, CECT 5947, CECT 4783, CECT 4782, CECT 4267, CECT 4076 checking:
(a) its amplification using the commercial kit from Applied Biosystems TaqMan® *E. coli* 0157:H7 for RT-PCR, and
(b) its viability and growth by plate count.

### Limit of detection in different samples

Artificial inoculation was carried out in the wash, rinse and centrifugal water of the processing line of fresh-processed lettuce with a cocktail of *E. coli* 0157 strains and the sensitivity of the plate count method and the RT-PCR commercial kit to detect the limit of detection in the various industrial process waters was determined. The wash, rinse and centrifugal water, was generated in the plant processing pilot plant using a water ratio of 10 times the volume of plant product and a concentration of sanitizer (sodium hypochlorite) in the wash tank of 100 parts per million (ppm). The rinsing process was carried out in water without sanitizers and, subsequently, the lettuce was centrifuged in the automatic centrifuge of the pilot plant.

The analysis by plate count and RT-PCR of the different waters from the processing line directly inoculated with *E. coli* 0157 to obtain a concentration of 10⁷ CFU/ml of water, showed that the sanitizer used (100 ppm sodium hypochlorite) in the wash water completely inactivated bacteria, obtaining counts of <1 CFU/ml (Table 1).

**Table 1. Comparison of detection of Escherichia coli 0157:H7 in various process waters of a fresh-processed line artificially inoculated with a high concentration (10⁷ CFU/ml) by plate count and by RT-PCR.**

| **Waters from the fresh-processed processing line** | **Plate count (CFU/ml)** | **RT-PCR (threshold cycle: C_{T})** |
|---|---|---|
| **Wash water** | **<1** | **25** |
| **Rinse water** | **1.68 x 10⁷** | **23.3** |
| **Centrifugal water** | **1.96 x 10⁷** | **24.2** |
| **Clean water** | **1.6 x 10⁷** | **23.9** |

However, the counts obtained in the rinse and centrifugal water resemble that of the added inoculum (Table 1). The RT-PCR analysis of water inoculated with a high concentration of *E. coli* (10⁷ CFU/ml) gave very similar results in all three types of water tested (wash, rinse and centrifuge). In the wash water the DNA of bacteria inactivated by the sanitizer was detected.

Is important to note that when the contamination of water occurs with a low concentration of bacteria (10² CFU/ml) the effectiveness of the method of detection by RT-PCR is different depending on the type of water if it is from washing, rinsing or centrifugation (Table 2).

**Table 2. Comparison of the detection of Escherichia coli 0157:H7 in various process waters of a fresh-processed line artificially inoculated with a low concentration (10² CFU/ml) by plate count and by RT-PCR.**

| **Waters from the fresh-processed processing line** | **Plate count (CFU/ml)** | **RT-PCR (threshold cycle :C_{T})** |
|---|---|---|
| **Wash water** | **<1** | **Negative** |
| **Rinse water** | **<1** | **36.0** |
| **Centrifugal water** | **1 X 10²** | **34.6** |

In fact, the wash water gave negative results by RT-PCR while the rinse and centrifugal water yielded positive results. Therefore, these results show that the detection of live or dead bacteria is much more efficient in centrifugal water where the C_{T} were lower.

The tests performed to find the limit of detection in the different waters showed that only in centrifugal water concentrations of 3 CFU/ml can be detected by RT-PCR, while in the wash and rinse water the results were negative (Table 3).

**Table 3. Limit of detection of Escherichia coli 0157:H7 in process water artificially inoculated with a very low concentration (3 CFU/ml).**

| **Waters from the fresh-processed processing line** | **Plate count (CFU/ml)** | **RT-PCR (threshold cycle C_{T})** |
|---|---|---|
| **Wash water** | <1 | Negative |
| **Rinse water** | <1 | Negative |
| **Centrifugal water** | 3 | 35.6 (50% negative and 50% positive) |
| **Wash water concentrated 100 times** | <1 | Negative |
| **Rinse water concentrated 100 times** | <1 | 35.4 (50% Negative and 50% positive) |
| **Centrifugal water concentrated 100 times** | 300 | 32.6 |

To improve the sensitivity in detecting bacteria in water where contamination is very low, a concentration of the samples by centrifugation (3,000 *g* for 10 minutes) could be carried out. This concentration process was effective in centrifugal water samples containing 3 CFU/ml since they gave higher counts and lower C_{T}s (Table 3). However, this process of concentration of the samples was not effective for detection in wash and rinse water (Table 3).

### EXAMPLE 2: Validation of the procedure by the analysis of contaminated product.

The industrial processing of contaminated samples of lettuce was simulated. To do this, iceberg lettuce artificially inoculated with two levels of E. *coli* 0157:H7 inoculum (10² CFU/g and 10⁵ CFU/g) was used. Once the lettuce has been inoculated, it underwent the process of cutting, washing and sanitation (100 ppm sodium hypochlorite aqueous solution, pH 6.5 and temperature of 4.5 °C), rinsing and subsequent centrifugation. Analyses of the different wash, rinse and centrifugal water, and the plant material after and before the washing processes, were carried out. Analyses were performed by plate count and RT-PCR. The wash, rinse and centrifugal water from the lettuce with low inoculum (10² cfu/g) was concentrated between 90 and 100 times by a centrifugation process prior to RT-PCR analysis.

Plate counts of lettuce inoculated with a high (1x10⁵ CFU/g) and a low (5x10² CFU/g) inoculum showed that at different process steps (washing, rinsing, centrifugation) totally eliminating contamination is not achieved, with the *E. coli* 0157:H7 concentration being 4x10⁴ and 83 CFU/g, respectively, in the final product (Table 4).

**Table 4. Detection by plate count and by RT-PCR of the contamination of E. coli 0157:H7 artificially inoculated with a high (10⁵ CFU/g) and a low (10² CFU/g) concentration bv analysis in lettuce and processing waters.**

| **Plate counts (CFU/g or CFU/ml)** | **High Inoculum** | **Low Inoculum** |
|---|---|---|
| **Raw material (before washing)** | 1.2 x 10⁵ | 5.0 x 10² |
| **Final product (after washing-centrifugation)** | 3.8 x10⁴ | 8.3 x 10 |
| **Wash water** | <1 | <1 |
| **Rinse water** | <1 | <1 |
| **Centrifugal water** | 3.8 x 10³ | 1.9 |

| **Counts by RT-PCR (C_{T})** | **High Inoculum** | **Low Inoculum** |
|---|---|---|
| **Wash water** | Negative | Negative |
| **Rinse water** | Negative | Negative |
| **Centrifugal water** | 30.6 | 35.8 |
| **Wash water concentrated 100 times** | | Negative |
| **Rinse water concentrated 100 times** | | Negative |
| **Centrifugal water concentrated 100 times** | | 32.7 |
| **RT-PCR Quantification of results (C_{T})** | **High Inoculum** | **Low Inoculum** |
| **Centrifugal water** | 1.4 x 10⁴ | 1.9 x 10² |
| **Centrifugal water concentrated 100 times** | | 2.5 x 10 |

Although the final food is still contaminated, the counts in the wash and rinse water were less than 1 CFU/ml, since the sanitizer used inactivated all the bacteria present in the water (Table 4). By contrast, in the centrifugal water the *E. coli* 0157:H7 count could be made both when the centrifuged lettuce had a high and a low inoculum.

The results obtained by RT-PCR again showed the greater suitability of centrifugal water compared to wash and rinse water for the detection of pathogens, since even though the final lettuce was still contaminated, detections in all waters were negative except in the centrifugal water (Table 4).

When the concentration of bacteria in water is very low (low inoculum) is necessary to carry out a concentration of the centrifugal water sample to make an indirect quantification of bacteria and not only detecting the presence or absence of the same.

### EXAMPLE 3: Preparation of the sample for analysis by RT-PCR.

From water collected from the various points outlined in Example 1, it is proceeded to the concentration of the sample:
1. The 250 ml of water are centrifuged at 3,400 *g* for 10 minutes and the pellet is resuspended in 1 ml of sterile distilled water.
2. Centrifuge at 13,000 *g* for 3 minutes the milliliter obtained in the previous step and the pellet is resuspended in 100 µl of Prepman Ultra simple preparation reagent (Applied Biosystems).
3. Stir and heat at 95-100 °C for 10 minutes.
4. Stir and centrifuge at 13,000 *g* for 3 minutes.
5. 100 µl of supernatant are taken into a sterile tube and stored at 4 °C or -20 °C.
6. A 1/10 dilution is performed by mixing 10 µl of the supernatant with of 90 µl of sterile nanopure water to obtain the DNA sample to be used in the RT-PCR reaction (see Example 4).

### EXAMPLE 4. Analysis of the Escherichia coli 0157:H7 concentration by RT-PCR.

The detection and analysis is performed by RT-PCR, namely with the Applied Biosystems 7500 Real-time PCR System equipment. For the detection of *E. coli* 0157, we used the commercial kit from Applied Biosystems TaqMan® *E. coli* 0157:H7 (which has an internal amplification control that prevents false negatives) following the protocol described by said company:

The RT-PCR reaction is prepared in 96-well plates *(ABl Prism 96-well Optical Reaction Plate)* in a final volume of 30 µl per well, as shown in Table 5.

**Table 5. Volumes of reagents for the preparation of the reaction.**

| **For each sample: 30 µl** | **For N samples** |
|---|---|
| 2X *Environmental Master Mix:* 15 µl | N x 15 µl |
| 10X *Target Assay Mix* 3 µl | N x 3 µl |
| DNA Sample: 12 µl | N x 12 µl |

Prepare the reaction mixture with the volumes indicated in Table 5, stir and dispense 30 µl of said mixture in each well of the 96-well plate. The plate is covered with adhesive covers and protected from light until the start of the reaction.

The RT-PCR program carried out consists of 10 minutes at 95 °C for polymerase activation, followed by 45 cycles of 15 seconds at 95 °C and 1 min at 60 °C.

The quantification standard curve fitting was carried out to correlate the measurements obtained by RT-PCR with the concentration of bacteria in the samples, obtained by plate count. The concentrations ranged from 2.2 ± 0.2 X10⁹ Colony Forming Units (CFU)/ml to 20 CFU/ml.

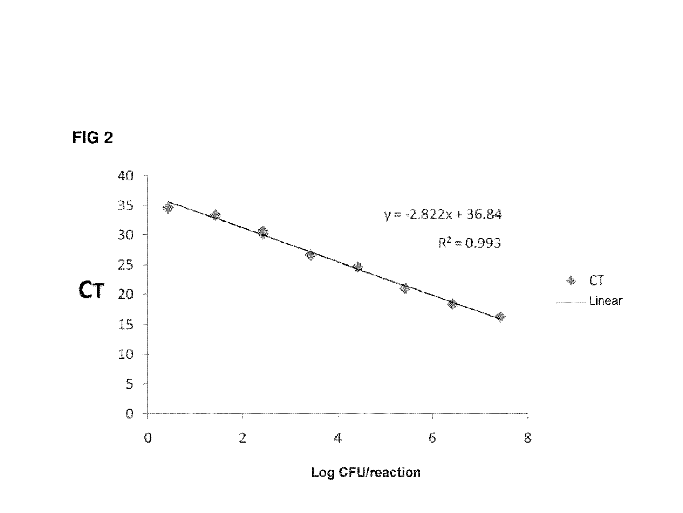

The RT-PCR kit used enabled a good fit in the quantification standard curve, although it lost the linearity when the concentration was 1 CFU/reaction. Therefore, quantification is possible up to 2.6 colonies per reaction although the method is able to detect up to 1 colony per reaction as shown in Figure 2.

### Extrapolation of the results of PCR to the concentration of pathogens and/or agents capable of causing the deterioration of the plant food

First, for carrying out the correlation of the measurements obtained by RT-PCR in centrifugal water samples after the concentration of pathogens and/or agents capable of causing the deterioration of plant food, the quantification standard curve fitting is carried out using as a DNA standard a cocktail of bacteria of the same species and serotype than those we want to detect in our samples (Fig. 2). Once our RT-PCR results of centrifugal water samples have been quantified, these results are correlated with the concentration of pathogens and/or agents capable of causing the deterioration in the raw material that has passed through the production line and in the elaborated food.

Based on our experimental results and by way of example, for processing iceberg lettuce, we observed that the concentration of *E. coli* 0157:H7 bacteria in centrifugal water is lower in 1 and 2 decimal logarithms than the concentration of bacteria in the plant product after and before the washing step, respectively (Table 4).

### Example 5: Example of time used to carry out the method of the invention.

### Sample collection

1- Industrial washing of the plant product: 0.5 to 2 minutes.
2 - Elimination by centrifugation/forced air tunnel, of the water from the plant surface: 0.5-1 minute.
3 - Collection of samples: for example, every hour 2 samples of 250 ml are taken.

### Time spent on analysis

1- Centrifugation of the sample in the laboratory: 10 minutes/3,400 *g*.
2 - DNA Extraction: 20 minutes.
3 - Preparation of the RT-PCR reaction: 5 minutes.
4 - Processing in the RT-PCR equipment: 1 hour.

Thus, in less than 2 hours the results of detection and analysis of potentially pathogenic agents in the water samples from plant active drainage or drying are obtained.

## Claims

1. A method for the detection of potentially pathogenic agents and/or agents capable of causing the deterioration of the plant in a plant sample, representative of at least one batch of plant product, **characterized in that** it comprises the following steps:
a. concentration of a plant sample by centrifugation,
b. analysis of the presence or the amount of potentially pathogenic agents in the concentrated sample obtained in (a).

2. The method according to the preceding claim, where the plant sample comprises water from the industrial washing of the plant.

3. The method according to the preceding claim, where the plant sample is obtained during the process of active drainage or drying of the plant.

4. The method according to the preceding claim, where the process of active drainage or drying of the plant is carried out by centrifugation or forced cold air tunnel.

5. The method according to any of the preceding claims, where the centrifugation of step (a) is carried out in one or more than one steps of centrifugation sequentially.

6. The method according to the preceding claim, where the centrifugation of step (a) is carried out in two sequential steps.

7. The method according to any of the preceding claims, where the centrifugation of step (a) allows concentrating the sample between 100 and 15,000 times.

8. The method according to any of the two preceding claims, where in the first centrifugation step the sample is concentrated between 100 and 500 times, and in the second centrifugation step, the sample is concentrated, in addition, between 5 and 25 times.

9. The method according to any of the preceding claims, where the centrifugation of step (a) is carried out at a speed of 2,000 *g* and 20,000 *g* for a time between 2 and 25 minutes.

10. The method according to any of the four preceding claims, where in step (a) is carried out a first centrifugation at a speed of between 2,000 *g* and 5,000 *g* for a time between 5 and 20 minutes and a second centrifugation at a speed of between 12,000 *g* and 20,000 *g* for a time between 2 and 5 minutes.

11. The method according to any of the preceding claims, wherein up to 3 colony forming units per gram of plant product are detected.

12. The method according to any of the preceding claims, wherein the duration of the two steps (a) and (b) is between 1.5 hours and 4 hours.

13. The method according to any of the preceding claims, wherein the duration of the two steps (a) and (b) is less than 2 hours.

14. A system for detection and analysis of potentially pathogenic agents and/or agents capable of causing the deterioration of the plant in a plant sample to carry out the method described in any one of claims 1 to 13, comprising an analyzer (9) for the detection and quantification of potentially pathogenic agents and/or agents capable of causing the deterioration of the plant, **characterized in that** it additionally comprises:
- a first tank (3) for collecting the water driven into said first tank from a container (1) for collecting the water removed from the plant surface during the active drainage or drying process through a conduit (2), by first driving means (4),
- a second tank (5) for collecting a sample of water, driven into said second tank from the first tank (3) by second driving means (6),
- centrifugation means (8) for concentrating the potentially pathogenic agents present in the water, and
- a control system (7) for regulating: the flow rate driven by the first driving means (4); the flow rate driven by the second driving means (6), and the centrifugation process, where the analyzer is adapted to analyze the centrifuged water.

15. The system for detection and analysis of potentially pathogenic agents and/or agents capable of causing the deterioration of the plant in a plant sample according to the preceding claim, **characterized in that** the first tank (3) and/or the second tank (5) comprise stirring means (10) for the homogenization of the water.

16. The system for detection and analysis of potentially pathogenic agents and/or agents capable of causing the deterioration of the plant in a plant sample according to any of the two preceding claims, **characterized in that** the first tank (3) and the second tank (5) comprise drainage systems (12) for draining said tanks (3) and (5).

17. The system for detection and analysis of potentially pathogenic agents and/or agents capable of causing the deterioration of the plant in a plant sample according to any of the three preceding claims, **characterized in that** it comprises a disinfection unit (11) to provide a disinfectant solution to the conduit (2) by third driving means (13).

18. The system for detection and analysis of potentially pathogenic agents and/or agents capable of causing the deterioration of the plant in a plant sample according to any of the four preceding claims, **characterized in that** at least one of the following elements: the stirring means (10) of the first tank (3) and the second tank (5); the drainage systems (12) for draining the first tank (3) and the second tank (5); the third driving means (13) for the application of the disinfectant solution of the disinfection unit (11) to the conduit (2), is regulated by the control system (7).

19. The system for detection and analysis of potentially pathogenic agents and/or agents capable of causing the deterioration of the plant in a plant sample according to any of the five preceding claims, **characterized in that** the centrifugation means (8) comprise two different sets of collecting tubes for sequential centrifugation of the samples in two different steps so that in the second step the pellet from the first step is centrifuged.
